# EUROPEAN PATENT APPLICATION

(11) **EP 4 613 182 A1**
(43) Date of publication of application: **10.09.2025**
(21) Application number: 23889094.1
(22) Date of filing: 07.11.2023
(51) Int. Cl.: A61B 5/02, A61B 5/022, A61B 5/00

(54) **BAND CAPABLE OF MEASURING ARTERIAL BLOOD**

(30) Priority: 07.11.2022 KR 20220146802; 03.11.2023 KR 20230150456
(71) Applicant: Lee, Sang Jin, Jinju-si, Gyeongsangnam-do 52717 (KR)
(72) Inventor: Lee, Sang Jin, Jinju-si, Gyeongsangnam-do 52717 (KR)
(74) Representative: Ter Meer Steinmeister & Partner
(86) International application number: PCT/KR2023/017710
(87) International publication number: WO 2024/101834

(57) **Abstract**

A band capable of measuring artery blood according to the present disclosure includes: a band unit elongated and wrapping around the wrist of a user; one or more pressing units configured on an inner surface of the band unit and configured to be able to compress any one of a radius portion and an ulna portion of the wrist of the user toward a center portion of the wrist; and a measuring unit provided on the band unit and measuring and collecting artery blood information with the pressing units pressing the radius portion and the ulna portion of the user, wherein the measuring unit is configured to be able to collect information of the artery blood at positions adjacent to a radius artery and an ulna artery of the user.

## Description

### BACKGROUND

### Technical Field

The present disclosure relates to a band capable of measuring arterial blood and more specifically, to a band capable of measuring arterial blood that can additionally measure arterial blood while compressing only the radial portion and the ulnar portion of the wrist toward the center of the wrist using a pad.

### Background Art

The wrist is formed by the radius and the ulna, which constitute the joints of the human arm, while the carpus is composed of eight carpal bones. The palm is formed by five metacarpophalangeal joints that enable rotation, flexion, and extension, and the fingers composed of multiple phalanges are formed to enable gripping objects.

These joint groups may sustain injuries due to repetitive and continuous overuse during hand-gripping exercises or activities, and in some cases, such injuries may not heal completely and may also cause persistent pain.

Accordingly, bands that are worn on the wrist of the human body to protect joints and bones have been devised and used.

In general, bands that require a protective function are worn on vulnerable joint areas of people along with an elbow guard, an ankle support, and a wrist support to protect these joints, and are worn by athletes of various sports, workers performing tasks, or children enjoying play for safety purposes.

Common bands according to the related art are formed in the type that wraps around the wrist and are configured in the type that applies uniform pressure around the entire wrist from the outer surface of the wrist. Through such wrist compression, the bands perform functions such as protecting the wrist joint and preventing excessive bending of the wrist.

However, such bands according to the related art merely perform the function of compressing the wrist and do not have any additional function to check health. Of course, recently developed products such as a smart band can check health by collecting the physiological information of users, but they do not have the function of providing protection through partial or overall compression of the wrist.

Of course, it may be partially possible to protect the body of a user and simultaneously collect physiological information by combining the technology of the compression bands or smart bands developed in the related art, but, this configuration can only collect information merely using venous blood.

Prior Art Document: Korean Utility Model No. 20-0389554

### SUMMARY OF THE DISCLOSURE

The present disclosure has been made in an effort to solve the problems of the related art described above, and is to provide a band capable of measuring arterial blood that can adjust compression load on the radius portion and the ulna portion and can additionally measure arterial blood while pressing the radial artery and ulnar artery through adjustment of the compression load when it is worn on the wrist of a user.

The objects of the present disclosure are not limited to the objects described above and other objects will be clearly understood by those skilled in the art from the following description.

A band capable of measuring artery blood according to an aspect of the present disclosure for achieving the objects described above includes: a band unit elongated and wrapping around a wrist of a user; one or more pressing units configured on an inner surface of the band unit and configured to be able to compress any one of a radius portion and an ulna portion of the wrist of the user toward a center portion of the wrist; and a measuring unit provided on the band unit and measuring and collecting artery blood information with the pressing units pressing the radius portion and the ulna portion of the user, wherein the measuring unit is configured to be able to continuously collect information of the artery blood at positions adjacent to a radius artery and an ulna artery of the user.

Further, the band unit may be elongated with a predetermined circumference and may selectively compress the radius artery and the ulna artery while compression load on the radius portion and the ulna portion by the pressing units is changed by selectively adjusting the circumference.

Further, the band unit may be adjusted in any one of a normal mode in which the pressing unit is maintained in a contact state while wrapping around the wrist of the user with a preset first pressure and a measuring mode in which the pressing units compress the wrist of the user with a second pressure higher than the first pressure to compress a portion of the radius artery and the ulna artery by at least partially compressing the skin of the user.

Further, a portion of the band unit in a longitudinal direction may be bent to overlap, so the entire circumference may be adjusted.

Further, the band unit may include Velcro at a portion in the longitudinal direction and may be configured such that a portion of the overlapping portion is selectively coupled.

Further, the band unit may include: a strip that is elongated and warps around a portion of the wrist of the user; and a coupling body having a predetermined area, having both end portions of the strip coupled to both end portions thereof, and enabling at least any one of both end portions of the strip to be coupled or separated by operation of a user.

Further, the strip may include: a first band having a predetermined length and coupled at the other end portion to a side of the coupling body; a second band having a shape similar to the first band, disposed to face the first band, and coupled at the other end portion to the other side of the coupling body; and a connection unit rotatably coupled at both end portions to one end portion of the first band and the second band, respectively, thereby connecting the first band and the second band.

Further, insertion guide holes may be formed at both ends portions of the coupling body so that the other end portions of the first band and the second band can be inserted therein, respectively, an operating unit that can be operated by the user may be mounted at a center portion of the coupling body, and the operating unit may be formed such that an insertion depth of the first band and the second band in the insertion guide holes can be adjusted.

Further, a pair of connection wires may be through-coupled to the other end portion of the first band and the second band and connected to the operating unit, respectively, and the connection wires may be drawn by operation of the operating unit, so the first band and the second band are inserted and coupled into the insertion guide holes.

Further, the pressing units may include a first pressing portion protruding from the band unit toward the wrist of the user and compressing any one of the radius portion or the ulna portion and a second pressing portion protruding from the band unit at a position spaced from the first pressing portion and compressing the other one of the radius portion or the ulna portion, and the pressing units may be formed not to compress the wrist except for the radius portion and the ulna portion.

Further, the first pressing portion and the second pressing portion may be curved to correspond to the shape of the wrist on a contact surface and may be formed to have a preset length in the longitudinal direction of the band unit.

Further, an end portion of the first pressing portion and the second pressing portion in a longitudinal direction may be elongated relatively longer to protrude toward the wrist of the user, and may compress the radius artery or the ulna artery by compressing the skin of the user in the measuring mode.

Further, the first pressing portion and the second pressing portion may be coupled to be movable in a predetermined section in the longitudinal direction of the band unit.

The band capable of measuring artery blood for achieving the objects of the present disclosure has the following effects.

First, the present disclosure is worn on the user's wrist and applying the radius and the ulna through the pressing units while simultaneously measuring the artery blood of the user with a separate measuring unit, so that it is possible to easily check the health condition.

Second, when the band is worn on the user's wrist, the compression intensity to the wrist is adjusted by adjusting a normal mode and a measuring mode, and the radius artery and the ulna artery are compressed in the measuring mode, so that it is possible to collect preset information through artery blood that cannot be measured in the related art, which provides the advantage that it is possible to collect more accurate information than information collection using veins in the related art.

The effects of the present disclosure are not limited to those described above and other effects not stated herein may be made apparent to those skilled in the art from the claims.

### BRIEF DESCRIPTION OF THE DRAWINGS

FIG. 1 is a view schematically showing the configuration of a band according to a first embodiment of the present disclosure;
FIG. 2 is a view showing the state in which the band of FIG. 1 is removed from a wrist;
FIG. 3 is a view showing a side of the band of FIG. 1;
FIG. 4 is a view showing the state in which the lower end portions of a first pressing portion and a second pressing portion are formed to protrude on the band of FIG. 1;
FIG. 5 is a view showing the state in which a pressing unit further includes a separate supporting member in the band of FIG. 1;
FIG. 6 is a view schematically showing the configuration of a band according to a second embodiment of the present disclosure;
FIG. 7 is a view showing the state in which the band of FIG. 6 is removed from a wrist;
FIG. 8 is a view showing switching to a wearing mode and a measuring mode according to the worn state of the band of FIG. 6;
FIG. 9 is a view showing the internal structure of an operating unit of the band of FIG. 6; and
FIG. 10 and FIG. 11 are views showing a structure in which the position of the pressing unit is adjusted in the band of FIG. 6.

### DETAILED DESCRIPTION

Hereafter, a preferred embodiment that can achieve the objects of the present disclosure is described in detail with reference to the accompanying drawings. In the description of the embodiment, the same components are given the same names and reference numerals and additional descriptions thereof are omitted.

Further, it should be noted that when embodiments of the present disclosure are described, components having the same functions are only given the same names and reference numbers and are not substantially completely the same as the related art.

Further, the terms used in embodiments of the present disclosure are used only to describe specific embodiments and are not intended to limit the present disclosure. Singular forms are intended to include plural forms unless the context clearly indicates otherwise.

It will be further understood that the terms "comprises" or "have" used in embodiments of the present disclosure specify the presence of stated features, numerals, steps, operations, components, parts, or a combination thereof, but do not preclude the presence or addition of one or more other features, numerals, steps, operations, components, parts, or a combination thereof.

### First Embodiment

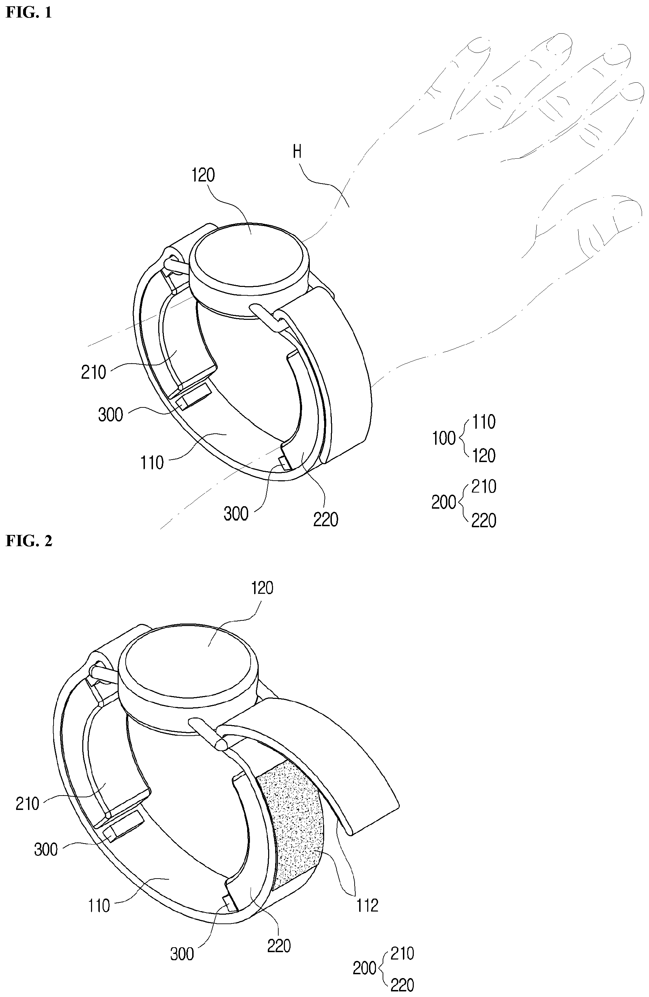

First, the configuration of a band capable of measuring artery blood according to a first embodiment of the present disclosure is described with reference to FIG. 1 to FIG. 3.

As shown in the figures, the band capable of measuring artery blood according to a first embodiment of the present disclosure is worn on the wrist H of a user and can stably protect the wrist H without side effects such as hand numbness, and mainly includes a band unit 100, a pressing unit 200, and a measuring unit 300.

The band unit 100 is formed long in a general band shape and is configured to wrap around the user's wrist H. In this case, the band unit 100 is formed in a ring shape to be at least partially separated or coupled or partially contracted and expanded around the circumference, and is configured to be able to worn on the user's wrist H.

In detail, the band unit 100 is made of a material such as rubber, fabric, synthetic resin, or the like, and is partially removed and separated along the circumference, so that, as shown in FIG. 2, a user can wear or separate the band unit on or from the wrist H.

In the present disclosure, the band unit 100 mainly includes a coupling body 120 and a strip 110, and the strip 100 and the coupling body 120 are connected to each other, so that a ringshaped ring is formed. The strip 100 is formed in a ring shape with a single layer or multiple layers and is configured to wrap around the user's wrist H together with the coupling body 120. In this case, the coupling body 120 may be configured to include a separate ring and at least any one of both longitudinal ends of the strip 110 is bent and fixed through the ring, so that the connection state can be removed or fixed.

In this case, the band unit 100 includes a separate Velcro 112 in a portion in the longitudinal direction and is configured such that a portion of the overlapping portion can be selectively coupled and fixed.

Further, the overall circumference of the strip 110 is adjusted in accordance with the length of the overlapping portion passing through the ring, so that the strip can be adjusted to have a circumference corresponding to the user's wrist H or can adjust pressure for compressing the user's wrist H.

In this embodiment, the strip 110 is formed long to have a predetermined circumference, and the compression load that is applied to the radius H1 portion and the ulna H portion of the user by the pressing unit 200 to be described below can be changed by selectively adjusting the circumference. Further, it is possible to selectively compress the radius H1 artery and the ulna H2 artery by adjusting the compression load of the pressing unit 200 through the strip 110.

As described above, the strip 110 is formed in a simple band type, as shown in the figures, and is configured to be coupled to the coupling body 120 or to be fixed by the Velcro 120 or the button through the separate ring to surround the user's wrist H.

Meanwhile, the coupling body 120 is disposed at a position facing the connection unit 130 and both end portions of the strip 110 are rotatably coupled to both sides.

In the present disclosure, the coupling body 120 has a predetermined area and both end portions of the strip 110 are rotatably coupled to both sides, respectively, and at least any one of both ends of the strip 110 is coupled or separated by operation of a user.

In detail, the coupling body 120 forms a ring shape together with the strip 110 and is configured to wrap around the user's wrist, and is supported such that the strip 110 may be connected to selectively adjust the circumference.

In this case, the coupling body 120 has a disc or polygonal plate shape, both longitudinal ends of the strip 110 are rotatably coupled to both end portions facing each other along the circumference, and at least any one side of the strip 110 is adjusted such that the circumference is adjusted while warping around the user's wrist H.

In this embodiment, the coupling body 120 is formed in a disc shape and has the rings formed at both end portions, and both ends of the strip 110 are rotatably coupled, respectively.

In this case, the coupling body 120 may include a control unit or a display unit such as a separate substrate or display, and may additionally include a battery, or the like, so that it is possible to record and analyze the information of artery blood in cooperation with the measuring unit 300 to be described below.

As described above, the band unit 100 according to the present disclosure includes the strip 110 and the coupling body 120 and is configured to wrap around the user's wrist H, and also can adjust the degree of compressing the user's wrist H by adjusting the circumference of the strip 110.

Next, the pressing unit 200 is configured as one or more on the inner surface of the band unit 100 and is configured to be able to compress any one of the radius H1 portion and the ulna H2 portion of the user's wrist H toward the center of the wrist H.

In detail, the pressing unit 200 is configured as one or more on the inner surface of the strip 110 and disposed to protrude toward the wrist H, and they each independently press the skin adjacent to the radius H1 portion and the ulna H2 portion of the user's wrist H.

In this case, the pressing unit 200 is configured as at least one or more pads and each of them independently protrudes on the inner surface of the strip 110 for pressing.

In this embodiment, the pressing unit 200 includes a first pressing portion 210 protruding toward the wrist H and a second pressing portion 220 protruding toward the wrist H from the second pressing portion, and the first pressing portion 210 and the second pressing portion 220 are configured to compress the ulna H2 portion and the radius H1 portion, respectively, without compressing the wrist H at other portions. In this case, the first pressing portion 210 and the second pressing portion 220 have a length relatively smaller than the strip 110, are formed such that a surface coming in contact with the wrist H is curved to correspond to the shape of the wrist H, and have a preset length in the longitudinal direction of the strip 110.

Further, as the length of the strip 110 coupled to the coupling body 120 to be described below is adjusted, it is possible to compress a portion of the radius H1 artery and the ulna H2 artery in a normal mode in which the pressing unit 200 maintains a contact state while wrapping around the user's wrist H with a first pressure or in a measuring mode in which at least a portion of the pressing unit 200 partially strongly compresses the skin of the user by compressing the wrist H with a second pressure relatively higher than the first pressure.

In this case, the first pressing portion 210 and the second pressing portion 220 are disposed in the same shape to be symmetric left and right, and may be formed such that an end portion in the longitudinal direction protrudes relatively longer.

As described above, the pressing unit 200 includes the first pressing portion 210 and the second pressing portion 220 and can press the radius H1 portion and the ulna H2 portion when a user wears the band on the wrist H through the strip 110.

Meanwhile, the measuring unit 300 is provided on at least any one of the coupling body 120 or the strip 110 and collects preset artery blood information by measuring artery blood of a user with the pressing unit 200 pressing the radius H1 portion and the ulna H2 portion of the user.

In detail, the measuring unit 300 is disposed on the strip 110 toward the user's wrist H and collects artery blood information by measuring artery blood selectively through the radius H1 artery and the ulna H2 artery of the user. In this case, the measuring unit 300 collects preset artery blood information by measuring artery blood in a non-contact type similarly to the method generally published.

In this case, the present disclosure is configured to collect information through artery blood rather than veins unlike the general technology, wherein the first pressing portion 210 and the second pressing portion 220 each compress the radius H1 artery and the ulna H2 artery of a user in the normal mode or the measuring mode, so that it is possible to measure artery blood.

In the first embodiment of the present disclosure, the measuring unit 300 is disposed on the strip 110 at a position adjacent to the radius H1 artery and the ulna H2 artery and is configured in a pair, and measures the artery blood of a user in a non-contact optical type toward the radius H1 artery and the ulna H2 artery of the user at the positions adjacent to the first pressing portion 210 and the second pressing portion 220. In this case, the measuring unit 300 may be configured in plural and may also be configured so that each of them additionally collects information through not only the radius H1 artery and the ulna H2 artery but also veins.

As described above, the measuring unit 300 additionally switches to the measuring mode from the normal mode with the first pressing portion 210 and the second pressing portion 220 pressing the radius H1 portion and the ulna H2 portion of a user, and presses the radius H1 artery and the ulna H2 artery, whereby it is possible to collect meaningful information from the radius H1 artery and the ulna H2 artery.

As described above, the band capable of measuring arterial blood according to the present disclosure is configured to be able to be worn on the user's wrist H, including the band unit 100, the pressing unit 200, and the measuring unit 300, and can be operated in the release mode, normal mode, and measuring mode in accordance with operation by a user, and the pressing units 200 can collect preset information by measuring each measure artery blood in accordance with the degree of pressing the radius H1 artery and the ulna H2 artery, depending on the normal mode or the measuring mode.

In general, it is possible to collect relatively accurate information from the arteries in comparison to the veins, so the arteries should accompany for a precise examination. However, since the arteries are positioned deep inside the skin of users, compression itself is relatively difficult, so, in practice, doctors, not nurses, directly perform measurement in the medical field.

However, the band capable of measuring arterial blood according to the present disclosure can collect preset information through the radius H1 artery and the ulna H2 artery by temporarily compressing arteries of the use simply without risk.

That is, it is possible to collect preset information through artery blood, which cannot be measured in the related art, by compressing the radius H1 artery and the ulna H2 artery in the measuring mode, which has the advantage that it is possible to collect accurate information in comparison to information collection using veins in the related art.

Further, in the normal mode, it is possible to measure information of the artery blood of a user and continuously perform measurement, so it is possible to collect artery blood information by continuously sensing variation of the electrocardiogram or blood pressure of a user.

In this case, it is possible to collect information by measuring the artery blood of a user in both of the normal mode or the measuring mode in the present disclosure, but the radius H1 artery and the ulna H2 artery of a user may be excessively compressed in the measuring mode, so it is preferable to apply the measuring mode only for short time.

In addition, a separate transmission/reception unit may be provided in the coupling body 120 and may be configured to transmit/receive the artery blood information of a user to/from the outside while collecting the artery blood information and to manage the health condition of a user in real time in cooperation with specialized institutions.

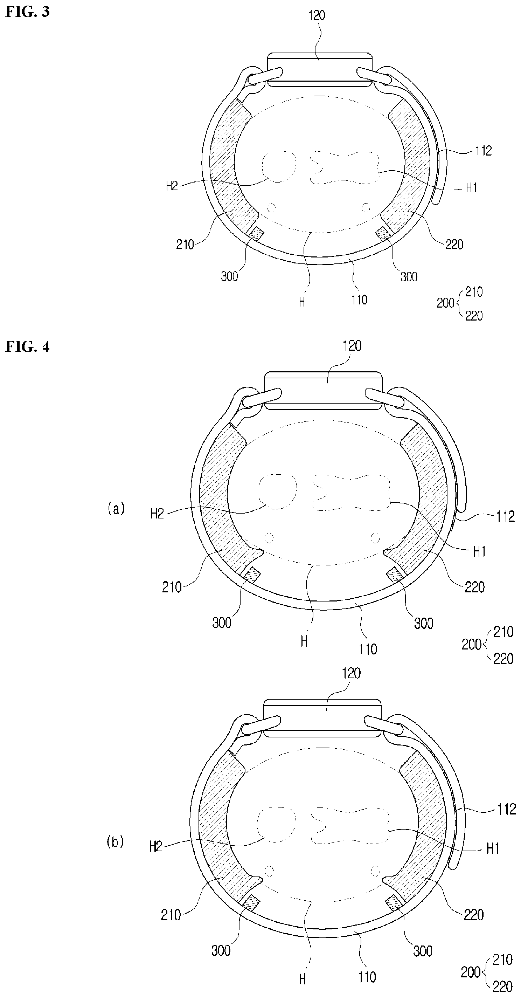

Next, a modification of the pressing unit 200 according to the first embodiment of the present disclosure is described hereafter with reference to FIG. 4.

FIG. 4 is a view showing the state in which the lower end portions of the first pressing portion 210 and the second pressing portion 220 are formed to protrude on the band of FIG. 1.

Referring to the figure, the basic configuration of the pressing unit 200 is substantially the same as the form described above, but some configurations of the first pressing portion 210 and the second pressing portion 220 are different. In detail, a longitudinal end portion of the first pressing portion 210 and the second pressing portion 220 protrudes relatively longer toward the use's wrist H and selectively additionally compresses the user's wrist H.

In this embodiment, as shown in FIG. 4A, the first pressing portion 210 and the second pressing portion 220 protrude by a predetermined length with a sharp longitudinal lower end portion, and compress the radius H1 portion and the ulna H2 portion of a user in the same way with the other portions in the normal mode.

However, as shown in FIG. 4B, in the measuring mode, the length of the strip 110 is reduced and simultaneously the first pressing portion 210 and the second pressing portion 220 additionally compress the radius H1 portion and the ulna H2 portion. In this case, the protruding portions of the first pressing portion 210 and the second pressing portion 220 increase the compression force at the user's wrist H, so strong compression is partially applied to the skin of the user.

In this case, the protruding portions of the first pressing portion 210 and the second pressing portion 220 compress the radius H1 artery and the ulna H2 artery while compressing a portion of the skin of the user, and accordingly, it is possible to compress arteries that cannot be compressed by simple compression.

That is, since the longitudinal end portions of the first pressing portion 210 and the second pressing portion 220 protrude, it is possible to more stably compress the radius H1 artery and the ulna H2 artery in the measuring mode.

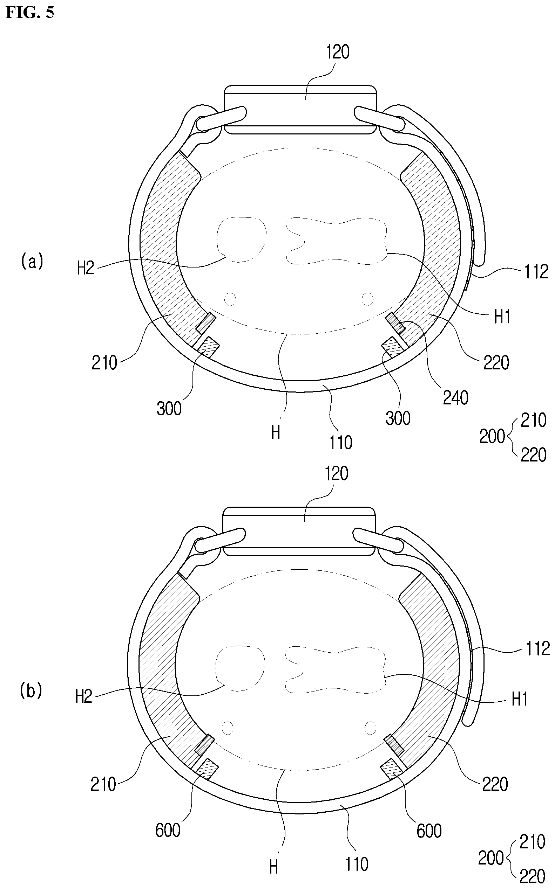

Next, a modification of the band according to the present disclosure is described hereafter with reference to FIG. 5.

FIG. 5 is a view showing the state in which the pressing unit 200 further includes a separate supporting member in the band of FIG. 1.

Referring to the figure, it is formed in a similar type to the embodiment described above, but the pressing unit 200 further includes a separate supporting member therein.

In detail, the first pressing portion 210 and the second pressing portion 220 further include a separate supporting member elongated in the thickness direction and protruding at the lower end portion in the longitudinal direction, and the supporting member is made of a material with relatively lower contractility than the first pressing portion 210 and the second pressing portion 220.

The first pressing portion 210 and the second pressing portion 220 of the present disclosure protrude from the first band 114 and the second band 116 to compress the radius H1 portion and the ulna H2 portion of a user and are configured to be partially contracted when compressed by compression force by performing compression with elasticity at a predetermined level.

In this case, if the supporting member is disposed in the first pressing portion 210 and the second pressing portion 220, contraction in the corresponding regions relatively decreases, which means that rigidity is increased by the supporting member and the corresponding regions can be relatively strongly compressed when the user's wrist H is compressed.

Accordingly, the supporting member according to the present disclosure is disposed in a way of protruding at a longitudinal end of the first pressing portion 210 and the second pressing portion 220, so that the supporting member compresses the radius H1 portion and the ulna H2 portion of a user with pressure similar to the other regions in the normal mode.

However, in the measuring mode, the pressing force of the first pressing portion 210 and the second pressing portion 220 is increased and accordingly, the contraction amount at portions adjacent to the supporting member decreases, and simultaneously, the radius H1 artery and the ulna H2 artery portions are pressed by partially compressing the skin of the user.

That is, the supporting member is provided at the longitudinal end portion of the first pressing portion 210 and the second pressing portion 220, and can press the radius H1 artery and the ulna H2 artery simultaneously with minimizing contraction at the corresponding regions in the measuring mode.

As described above, a band capable of measuring arterial blood according to the first embodiment of the present disclosure was described, in which the degree of compressing the user's wrist H by the pressing units 200 is adjusted by adjusting the length of the strip 110 and the pressing units 200 partially press the radius H1 artery and the ulna H2 artery of the user, so it is possible to stably collect information of the artery blood.

### Second Embodiment

Next, a second embodiment according to the present disclosure is described.

The band according to the second embodiment of the present disclosure is configured in a partially similar type to the first embodiment described above, but has different configuration from the configuration of the band unit 100.

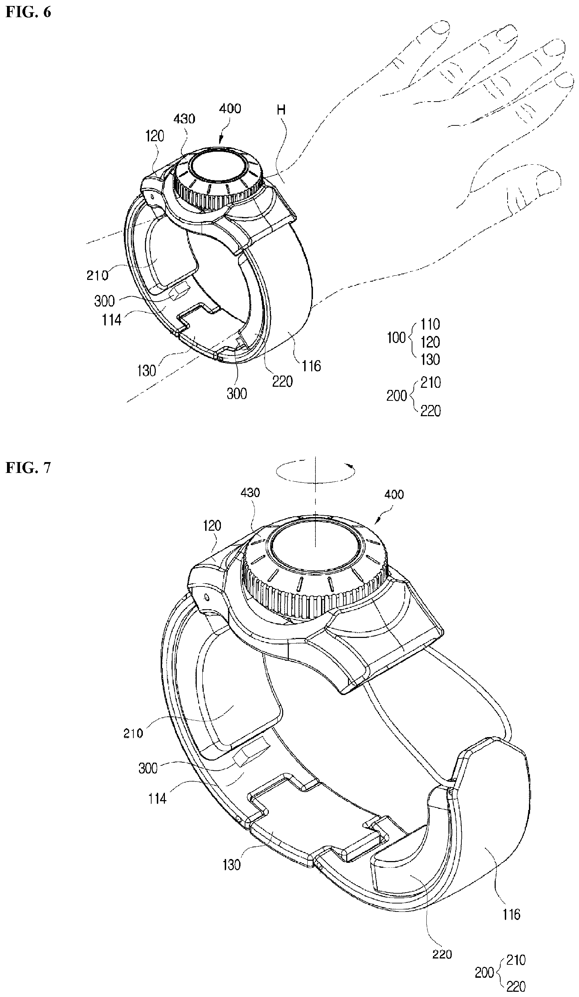

First, the configuration of the second embodiment of the present disclosure is described hereafter with reference to FIG. 6 to FIG. 9.

FIG. 6 is a view schematically showing the configuration of the band according to the second embodiment of the present disclosure, and FIG. 7 is a view showing the state in which the band of FIG. 6 is removed from a wrist H.

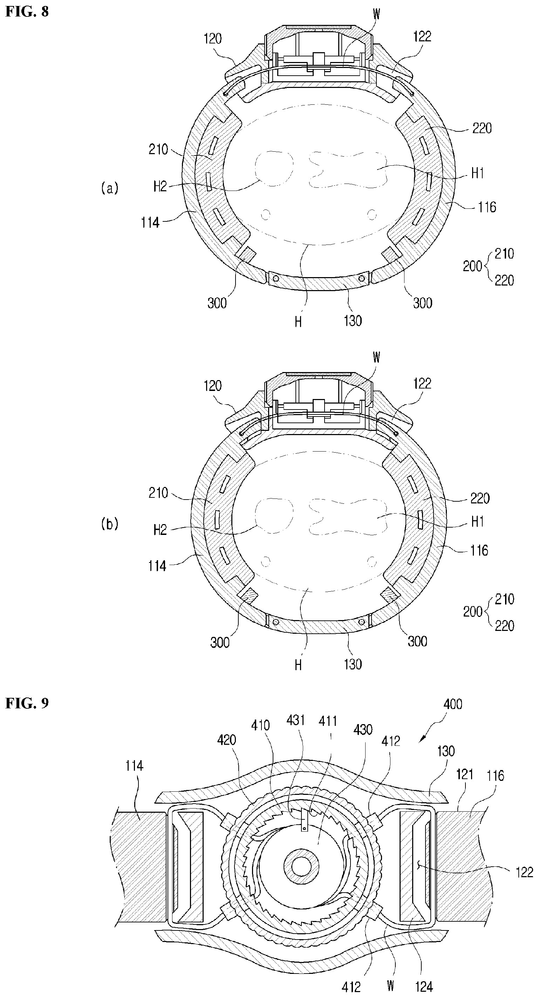

Further, FIG. 8 is a view showing switching to a wearing mode and a measuring mode according to the worn state of the band of FIG. 6, and FIG. 9 is a view showing the internal structure of an operating unit 400 in the band of FIG. 6.

Referring to the figures, the band unit 100 is adjusted in circumference in a way of wrapping around the user's wrist H and has the pressing unit 200 and the measuring unit 300 on the inner surface. Further, the band unit 100 includes a strip 110 and a coupling body 120, and unlike the first embodiment, the configurations of the strip 110 and the coupling body 120 are different.

In detail, the strip 110 forms a ring shape by being coupled to the coupling body 120 and, unlike the first embodiment, a plurality of members are continuously connected in the longitudinal direction.

The strip 110 according to the second embodiment of the present disclosure mainly includes a first band 114, a second band 116, and a connection unit 130, and the first band 114 has a predetermined length and other end portion thereof is rotatably coupled to the coupling body 120. Further, the second band 116 has a shape similar to the first band 114 and is disposed to face the first band, and another end portion thereof is rotatably coupled to the coupling body 120.

That is, the first band 114 and the second band 116 each have a predetermined length and are configured to wrap around the wrist of a user by being rotatably coupled to both sides around the coupling body 120.

In this case, the first band 114 and the second band 116 are formed in a shape in which the inner surface is concavely recessed to be able to wrap around the wrist H, and the pressing units 200 are mounted at a side on the inner surfaces to be able to each compress the radius H1 portion and the ulna H2 portion of the wrist H toward the center of the wrist H.

The connection unit 130 is one of components connecting the first band 114 and the second band 116, and both end portions are rotatably coupled to one end portions of the first band 114 and the second band 116, respectively, thereby connecting the first band 114 and the second band 116.

In detail, the first band 114 and the second band 116 are disposed such that the pressing units 200 disposed on them, respectively, compress the radius H1 and ulna H2 portions of the wrist H toward the center of the wrist H while being adjacent to them. In this case, the connection unit 130 is configured to maintain a connection state by being rotatably coupled to one ends of the first band 114 and the second band 116 and to be able to support them without separating even though the pressing units 200 presses the radius H1 and ulna H2 portions by the coupling body 120 to be described below.

As described above, the strip 110 includes the first band 114, the second band 116, and the connection unit 130, and as shown in the figures, is formed in a simple band type, and is coupled to the coupling body 120 such that the circumference is selectively adjusted.

Meanwhile, the coupling body 120 according to the second embodiment, unlike the first embodiment described above, includes separate connection wire W and operating unit 400 therein, is connected with the strip 110, and is configured such that the length can be selectively adjusted when it is coupled to the strip 110.

In detail, the coupling body 120 is disposed at a position facing the connection unit 130, and the other end portions of the first band 114 and the second band 116 are rotatably coupled to both sides thereof, respectively. In this case, the coupling body 120 is adjusted such that at least any one of the first band 114 and the second band 116 is coupled or separated by operation of a user, so that the user can wear or release the band according to the present disclosure.

In the present disclosure, the coupling body 120 is formed such that the first band 114 and the second band 116 are rotatably coupled or separated to or from one side, and in this case, the coupling state of the first band 114 and the second band 116 can be adjusted by operation of a user such that the compression load on the radius H1 portion and the ulna H2 portion by the first band 114 and the second band 116 can be adjusted.

Accordingly, in the present disclosure, when the first band 114 and the second band 116 are coupled to the coupling body 120, the coupling length is adjusted, and the degree of compressing the wrist of a user by the first band 114 and the second band 116 is adjusted, so that it can be adjusted into any one of the release mode, the normal mode, and the measuring mode.

In detail, insertion guide holes 122 are formed at end portions of the coupling body 120, respectively, such that the other end portion of the first band 114 can be inserted and coupled in an end portion of the coupling body 120 and the other end portion of the second band 116 can be inserted and coupled in the other end portion thereof, and the operating unit 400 that can be operated by a user is provided at the center portion of the coupling body 120. Further, the operating unit 400 is formed such that the insertion depth of the first band 114 and the second band 116 in the insertion guide holes 122 can be adjusted.

In this case, separate connection wires W are coupled to the operating unit 400 of the coupling body 120, respectively, through the other end portions of the first band 114 and the second band 116, and a pair of connection wires W can be simultaneously drawn by operation of the operating unit 400 such that the first band 114 and the second band 116 are inserted and coupled into the insertion guide holes 122.

That is, the connection wires W are independently connected to the first band 114 and the second band 116, so that insertion of the other end portions in the insertion guide holes 122 is adjusted by the operating unit 400, and can be adjusted into any one of the release mode, the normal mode, and the measuring mode, depending on whether insertion is performed and the insertion depth.

According this configuration, by drawing and releasing the connection wires W by operating the operating unit 400 of the coupling body 120, as shown in FIG. 7, the first band 114 and the second band 116 can be disposed in the release mode in which they are separated from the coupling body 120. On the contrary, by drawing the connection wires W by operating the operating unit 400, as shown in FIG. 8, the first band 114 and the second band 116 can be coupled to the coupling body 120.

The band capable of measuring artery blood always maintains the overall ring shape even though the first band 114 and the second band 116 are separated from the coupling body 120 because the connection state is maintained through the connection wires W, and as shown in FIG. 7, the band can be worn on the wrist H, as shown in FIG. 6, by positioning the wrist H in the direction passing through the internal space of the band in an open state, in which the second body 116 is separated from the coupling body 120, as shown in FIG. 7, and then drawing the connection wires W by operating the operating unit 400.

In this case, it is required to apply different kinds of bands, depending on the thickness of the wrist H of the user, and in this case, it is possible to differently adjust the diameter of the internal space of the band capable of measuring artery blood by manufacturing the first band 114 and the second band 116 in the same size and manufacturing and coupling only the connection unit 130 with a different length.

Further, the insertion depth of the second band 116 into the insertion guide hole 122 can be adjusted into the normal mode or the measuring mode by operation of the operating unit 400. In this case, in the case of the measuring mode, the compression load on the radius H1 portion and the ulna H2 portion by the first band 114 and the second band 116 is relatively increased, and in the case of the normal mode, the first band 114 and the second band 116 are relatively shallowly inserted into the insertion guide holes 122, so the compression load on the radius H1 portion and the ulna H2 portion is relatively decreased.

In more detail, FIG. 8A shows the state in which the first band 114 and the second band 116 are coupled to the coupling body 120 in the normal mode, and in this case, they are only partially inserted in the insertion guide holes 122.

The normal mode is the state in which the first pressing portion 210 and the second pressing portion 220 compress the user's wrist H with a first pressure, which is basic wearing type.

Further, FIG. 8B is the state in which the first band 114 and the second band 116 are fully inserted into the guide holes 122 and coupled in the measuring mode, in which the bands are tightened to more strongly compress the user's wrist H in comparison to the normal mode, and the first pressing portion 210 and the second pressing portion 220 intensively compress the radius H1 portion and the ulna H2 portion.

In this case, the measuring mode is the state in which the first pressing portion 210 and the second pressing portion 220 compress the user's wrist H with a second pressure higher than the first pressure, and in the measuring mode, the longitudinal ends of the first pressing portion 210 and the second pressing portion 220 partially compress the skin of the user, thereby additionally compressing the radius H1 artery and ulna H2 artery portions. Accordingly, it is possible to measure the artery blood of the user through the measuring unit 300 to be described below.

Meanwhile, the operating unit 400 includes a fixed body part 410 that is coupled and fixed to the coupling body 120 and has a ratchet gear 411 formed on the inner surface at the open upper end portion, a rotary body part 430 coupled to the fixed body part 410 to be movable up and down and has a stopper protrusion 431 formed on a side and engaged with the ratchet gear 411 to restrict rotation in one direction when it is moved downward, and in which the stopper protrusion 431 and the ratchet gear 411 are disengaged when it is moved upward, and a dial knob 420 coupled to the rotary body part 430 to move up and down and rotate integrally with the rotary body part 430 and formed to be operated by a user.

Further, the connection wires W through-coupled to the first band 114 and the second band 116 are connected to the rotary body part 430 to be simultaneously wound.

According to the operating unit 400 configured as described above, when the dial knob 420 is rotated in a first direction with the dial knob 420 pressed and moved downward, the rotary body part 430 is rotated with the dial knob 420, so the connection wires W are wound on the rotary body part 430.

Accordingly, the connection wires W are drawn to the coupling body 120, so that the first band 114 and the second band 116 are inserted and coupled into the insertion guide holes 122. In this state, rotation of the rotary body part 430 in the opposite direction is restricted because the stopper protrusion 431 is engaged with the ratchet gear 411 of the fixed body part 410, so that, as the dial knob 420 is further rotated, the first band 114 and the second band 116 are further deeply inserted into the insertion guide holes 122, and accordingly, the compression load on the radius H1 portion and the ulna H2 portion is increased and this state is maintained.

That is, when the first band 114 and the second band 116 are inserted into the insertion guide holes 122 to a predetermined depth by drawing the connection wires W by a preset length through the operation unit 400, the user's wrist H is compressed in the normal mode, and when the first band 114 and the second band 116 are fully inserted into the insertion guide holes 122 by additionally drawing the connection wires W, it is possible to compress the user's wrist W in the measuring mode.

On the contrary, in order to reduce the compression load on the radius H1 portion and the ulna H2 portion or separate the first band 114 and the second band 116 from the coupling body 120, it is possible to disengage the stopper protrusion 431 of the rotary body part 430 and the ratchet gear 411 of the fixed body part 410 by drawing upward the dial knob 420, and in this state, it is possible to rotate the first band 114 and the second band 116 in the direction in which they are separated from the coupling body 120 or rotate the dial knob 420 counterclockwise.

Meanwhile, wire guide portions 124 that guide the connection paths of the connection wires W such that the connection wires W of the first band 114 and the second band 116 are connected to the operating unit 400 in a way of winding on the rotary body part 430 may be formed at the insertion guide holes 122 of the coupling body 120.

The wire guide portions 124 may be formed to guide the connection wired W passing through the second band 116 to the edges of both sides of the insertion guide holes 122.

Further, wire inlets 412 that guide the through-paths of the connection wires W such that the connection wires W connected from the first band 114 and the second band 116 can be wound on the rotary body part 430 through them may be formed at the fixed body part 410. The wire inlets 412 may be formed at positions adjacent to the edges of both sides of the insertion guide holes 122 in a way of corresponding to the wire guide portions 124.

Since the connection wires W are maintained in the state in which they are disposed in predetermined paths by the wire guide portions 124 and the wire inlets 412, they can be stably maintained without problems such as twisting or entangling with each other even though the first band 114 and the second band 116 are coupled to or separated from the coupling body 120.

As described above, the band unit 100 according to the second embodiment of the present disclosure is configured in the type in which the coupling body 120 and the strip 110 are different from the first embodiment described above, so that the circumference of the strip 110 is adjusted by operating the operating unit 400, and accordingly, it is possible to adjust the degree of wrapping around and compressing the user's wrist H.

Next, the pressing unit 200 according to the second embodiment of the present disclosure, similarly to the first embodiment described above, includes the first pressing portion 210 and the second pressing portion 220 and at least one or more pressing portions are configured on the inner surface of the first band 114 and the second band 116, respectively, and disposed to protrude toward the wrist H.

In this case, the first pressing portion 210 and the second pressing portion 220 are configured to be able to compress at least one of the radius H1 portion and the ulna H2 portion of a user, respectively, toward the center.

In this case, the first pressing portion 210 and the second pressing portion 220 are disposed in the same shape to be symmetric left and right, and may be formed such that an end portion in the longitudinal direction protrudes relatively longer.

As described above, the pressing unit 200 includes the first pressing portion 210 and the second pressing portion 220 and can press the radius H1 portion and the ulna H2 portion when a user wears the band on the wrist H through the strip 110.

Further, the measuring unit 300, similarly to the first embodiment described above, is disposed to be adjacent to the radius H1 portion and the ulna H2 portion and the pressing unit 200 measures and collects information of the artery blood while pressing the user's wrist H.

In this case, as shown in the figures, the measuring unit 300 is composed of at least one of the first band 114 and the second band 116 or the connection unit 130 at positions adjacent to the radius H1 artery and the ulna H2 artery, and measures the artery blood of a user in a non-contact optical type toward the radius H1 artery and the ulna H2 artery of the user at positions adjacent to the first pressing portion 210 and the second pressing portion 220.

As described above, the band according to the second embodiment of the present disclosure fundamentally has a shape similar to the first embodiment, and is different in the configuration of the band unit 100, so it has a difference in the structure for coupling to the user's wrist H and the way of adjusting the length of the circumference.

However, in the band of the second embodiment, similarly to the first embodiment described above, the pressing units 200 also compress the user's wrist H at positions adjacent to the radius H1 artery and the ulna H2 artery, whereby it is possible to measure artery blood information.

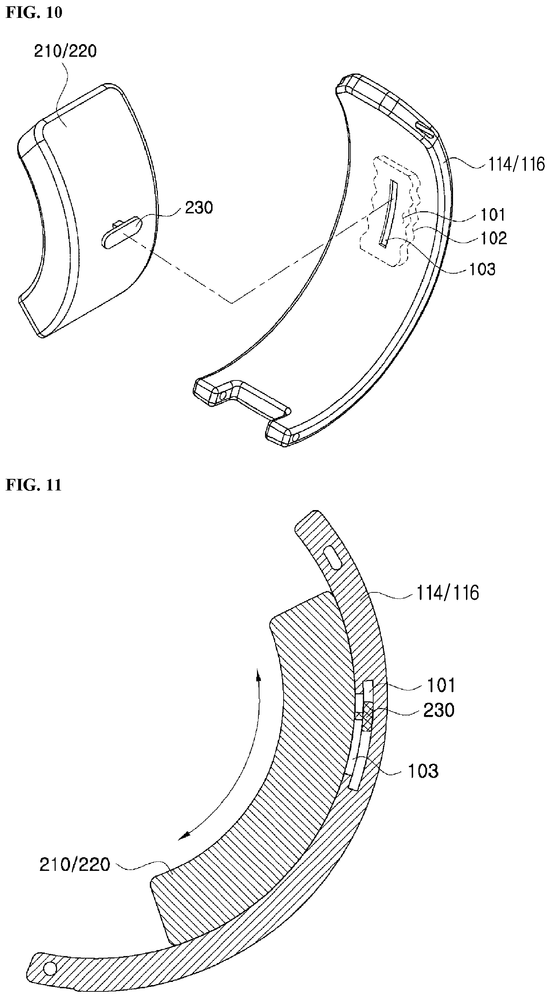

Next, the configuration of adjusting the position of the pressing units 200 of the band capable of measuring artery blood according to the second embodiment of the present disclosure is described hereafter with reference to FIG. 10 and FIG. 11.

As shown in the figures, the pressing units 200 may be mounted to be movable a predetermined section in the longitudinal direction of the first band 114 and the second band 116, and accordingly, it is possible to adjust the positions of the first pressing portion 210 and the second pressing portion 220, so that it is possible to accurately compress the radius and ulna H2 portions that may be different, depending on users.

In detail, when the band capable of measuring artery blood according to the present disclosure is worn on a wrist H, the first pressing portion 210 and the second pressing portion 220 are disposed to face each other and compress the radius H1 portion and the ulna H2 portion of the wrist H. In this case, since the shape of the wrist H and the positions of the radius H1 and the ulna H2 may be slightly different among individuals, the first pressing portion 210 and the second pressing portion 220 may fail to press the exact points, depending on users.

Accordingly, a user can position the first pressing portion 210 and the second pressing portion 220 at accurate positions by adjusting the positions by moving the first pressing portion 210 and the second pressing portion 220 to be suitable for himself/herself, and accordingly, it is possible to accurately compress not only the radius H1 and the ulna H2, but also the radius H1 artery and the ulna H2 artery.

In order to move the positions of the first pressing portion 210 and the second pressing portion 220, insertion protrusions 230 that can be inserted into the internal spaces of the first band 114 and the second band 116 may be formed on the contact surfaces of the first pressing portion 210 and the second pressing portion 220 with the first band 114 and the second band 116.

Further, pad accommodation portions 101 are formed on the first band 114 and the second band 116 so that the insertion protrusions 230 can be inserted and moved therein, and movement guide portions 102 are formed in the internal spaces of the pad accommodation portions 101 so that the insertion protrusions 230 can be moved by external force in an engaged state.

In this case, openings 103 may be longitudinally formed on the inner surfaces of the first band 114 and the second band 116 so that the insertion protrusions 230 of the first pressing portion 210 and the second pressing portion 220 are inserted therein. The insertion protrusions 230 are inserted and accommodated in the pad accommodation portions 101 through the openings 103 and can be longitudinally moved along the movement guide portions 102 disposed in the pad accommodation portions 101.

Further, a concave-convex portion is formed on the edge of the movement guide portions 102 and the insertion protrusions 230 may be configured to be separately moved by external force in an engaged state with the concave-convex portions.

According to this configuration, when a user wears the band capable of measuring artery blood, the user can adjust positions in this state by pushing or pulling the first pressing portion 210 and the second pressing portion 220 in the longitudinal direction, and accordingly, it is possible to more accurately compress the radius H1 portion and the ulna H2 portion.

Although preferred embodiments of the present disclose were described above, it would be apparent to those skilled in the art that the present disclosure may be achieved in other specific types without departing from the scope or spirit other than the embodiments described above. Accordingly, the embodiments described above should be considered as being illustrative rather than limiting, and accordingly the present disclosure may be changed within the range of the claims and the equivalent range without being limited to the above description.

## Claims

1. A band capable of measuring artery blood, comprising:
a band unit elongated and wrapping around a wrist of a user;
one or more pressing units configured on an inner surface of the band unit and configured to be able to compress any one of a radius portion and an ulna portion of the wrist of the user toward a center portion of the wrist; and
a measuring unit provided on the band unit and measuring and collecting artery blood information with the pressing units pressing the radius portion and the ulna portion of the user,
wherein the measuring unit is configured to be able to collect information of the artery blood at positions adjacent to a radius artery and an ulna artery of the user.

2. The band of claim 1, wherein the band unit is elongated with a predetermined circumference and can selectively compress the radius artery and the ulna artery while compression load on the radius portion and the ulna portion by the pressing units is changed by selectively adjusting the circumference.

3. The band of claim 2, wherein the band unit is adjusted in any one of a normal mode in which the pressing unit is maintained in a contact state while wrapping around the wrist of the user with a preset first pressure and a measuring mode in which the pressing unit compresses the wrist of the user with a second pressure relatively higher than the first pressure to compress a portion of the radius artery and the ulna artery by at least partially compressing the skin of the user.

4. The band of claim 2, wherein a portion of the band unit in a longitudinal direction is bent to overlap, so the entire circumference is adjusted.

5. The band of claim 4, wherein the band unit includes Velcro at a portion in the longitudinal direction and is configured such that a part of the overlapping portion is selectively coupled.

6. The band of claim 2, wherein the band unit includes:
a strip elongated and wrapping around a portion of the wrist of the user; and
a coupling body having a predetermined area, having both end portions of the strip coupled to both end portions thereof, and enabling at least any one of both end portions of the strip to be coupled or separated by operation of a user.

7. The band of claim 6, wherein the strip includes:
a first band having a predetermined length and coupled at the other end portion to one side of the coupling body;
a second band having a shape similar to the first band, disposed to face the first band, and coupled at the other end portion to the other side of the coupling body; and
a connection unit rotatably coupled at both end portions to the end portions of the first band and the second band, respectively, thereby connecting the first band and the second band.

8. The band of claim 7, wherein insertion guide holes are formed at both end portions of the coupling body so that the other end portions of the first band and the second band can be inserted therein, respectively,
an operating unit that can be operated by the user is mounted at a center portion of the coupling body, and
the operating unit is formed such that an insertion depth of the first band and the second band in the insertion guide holes can be adjusted.

9. The band of claim 8, wherein a pair of connection wires is through-coupled to the other end portion of the first band and the second band and connected to the operating unit, and
the connection wires are drawn by operation of the operating unit, so that the first band and the second band are inserted into and coupled to the insertion guide holes.

10. The band of claim 3, wherein the pressing unit includes a first pressing portion protruding from the band unit toward the wrist of the user and compressing any one of the radius portion or the ulna portion and a second pressing portion protruding from the band unit at a position spaced from the first pressing portion and compressing the other one of the radius portion or the ulna portion; and
the pressing unit is formed not to compress the wrist except for the radius portion and the ulna portion.

11. The band of claim 10, wherein the first pressing portion and the second pressing portion are curved to correspond to the shape of the wrist on a contact surface and are formed to have a preset length in the longitudinal direction of the band unit.

12. The band of claim 11, wherein an end portion of the first pressing portion and the second pressing portion in a longitudinal direction is elongated relatively longer to protrude toward the wrist of the user, and compress the radius artery or the ulna artery by compressing the skin of the user in the measuring mode.

13. The band of claim 11, wherein the first pressing portion and the second pressing portion are coupled to be movable in a predetermined section in the longitudinal direction of the band unit.
